(19)

Europäisches Patentamt
European Patent Office
Office européen des brevets

(11)  **EP 4 732 824 A1**

(12)  # EUROPEAN PATENT APPLICATION

(43) Date of publication:
**29.04.2026   Bulletin 2026/18**

(21) Application number: **25209097.2**

(22) Date of filing: **16.10.2025**

(51) International Patent Classification (IPC):
**A61K 8/24** (2006.01)     **A61K 8/365** (2006.01)
**A61K 8/86** (2006.01)     **A61Q 5/10** (2006.01)

(52) Cooperative Patent Classification (CPC):
**A61Q 5/10; A61K 8/24; A61K 8/365; A61K 8/86;**
A61K 2800/5922; A61K 2800/882

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **23.10.2024  IT 202400023667**

(71) Applicant: **Deco Hair S.p.A.**
**46040 Guidizzolo (MN) (IT)**

(72) Inventor: **PALVARINI, Franco**
**46040 Guidizzolo (MN) (IT)**

(74) Representative: **Botti & Ferrari S.p.A.**
**Via Cappellini, 11**
**20124 Milano (IT)**

(54)  **HAIR DYE ADDITIVE AND HAIR DYE CONTAINING SAID ADDITIVE**

(57)   The present invention relates to a hair dye additive comprising citric acid, phosphoric acid, polyethylene glycol (PEG), and at least one further acid selected from malic acid, lactic acid, succinic acid and combinations thereof, preferably malic acid. The invention also relates to a hair dye, in particular an oxidation dye comprising said additive and a method for the preparation of a mixture for hair dyeing comprising the mixture of a hair dye containing dyeing precursors, an oxidizer, and said additive.

Fig. 1

EP 4 732 824 A1

**Description**

Field of application

In its more general aspect, the present invention relates to products for hair dyeing.

In particular, the present invention relates to an additive for a hair oxidation dye and to a hair oxidation dye comprising said additive.

The present invention also relates to a method for the preparation of a mixture for hair dyeing comprising the mixing of a hair dye containing dyeing precursors, an oxidizer and said additive.

Prior art

As is well known, hair dyes are widely used to revive natural colour, lighten colour or restore white hair to its original colour.

In particular, among the most commonly used hair dyes are oxidation dyes, which lighten or darken hair colour, intensify or alter highlights thereof, while ensuring a long-lasting, persistent result. These dyes are generally produced in the form of cream, oil or gel products containing colourless substances, called precursors, which are transformed into dyes only after oxidation, i.e. only after mixing with an oxidizer.

Usually, the oxidizer used is hydrogen peroxide $H_2O_2$ which, in addition to participating in the formation of pigments, promotes the colouring process by exerting a bleaching action of the hair, i.e. destroying the melanin present in the hair, which is thus lightened and, therefore, more susceptible to colouring.

Precursors are usually organic compounds belonging to the benzene series in which amino groups $-NH_2$ or alcohol groups -OH are present, such as para-diaminobenzene, meta-dihydroxybenzene, and para-aminophenol.

A significant drawback of the above oxidation dyes is represented by their vapours, particularly during use.

Indeed, mixing the oxidiser and precursors leads to the development of ammonia which, vaporising almost instantly, causes the formation of a particularly irritating and foul-smelling gas/vapour.

This problem is particularly evident when one considers that this mixing is carried out by professional operators (e.g. hairdressers in a beauty centre or salon) on site and only at the time of colouring, so that the aforementioned vapours are largely inhaled by hairdresser and customers.

Moreover, the compound obtained by said mixing is left on the hair for about 30-40 minutes, forcing the customer to inhale large quantities of ammonia.

In an attempt to overcome these drawbacks, additives for hair dyes have been developed that are capable of combining with the free ammonia that develops as a result of mixing the oxidizer with dye precursors, transforming it into a soluble salt. In this way, a significant reduction in ammonia vapours from the mixture of dye precursors and oxidiser is achieved. An additive of this type is described, for instance, in EP2873411 patent application and comprises 30% to 50% citric acid, 30% to 50% phosphoric acid and 20% to 30% polyethylene glycol (PEG).

Additives for hair dyes, although known to help reduce ammonia vapour emissions from dyes, are not entirely satisfactory.

In this regard, there is a need to provide an additive for hair dye that, in addition to reducing ammonia vapour emissions as much as possible, also improves the application of the dye to the hair, in particular by providing better colour coverage and/or maintaining the integrity of the hair.

An object of the present invention is therefore to provide a hair dye additive that reduces the release of ammonia vapours from such dyes.

Another object of the present invention is to provide a hair dye additive that improves hair colour coverage and/or maintains hair integrity when used in combination with the dye.

A further object of the present invention is to provide a hair dye additive that does not interfere with the colour generated during use of the dye and therefore does not alter the colour of the hair.

Summary of the invention

The above objects are attained by a hair dye additive comprising citric acid, phosphoric acid, polyethylene glycol (PEG), and at least one further acid selected from malic acid, lactic acid, succinic acid, and combinations thereof.

Preferably, the hair dye additive according to the invention comprises citric acid, phosphoric acid, polyethylene glycol (PEG), and malic acid.

The above objects are also attained by a hair dye, in particular an oxidation dye comprising an additive as above.

The above objects are also attained by a method for the preparation of a hair dye, in particular an oxidation dye comprising an additive as above, the method comprising the steps of:

- providing a hair dye containing dyeing precursors,

- mixing the above described additive with said dye.

The above objects are also attained by a method for the preparation of a mixture for hair dyeing, the method comprising mixing an oxidation dye, an oxidizer and said additive.

As will be more evident in the experimental part of this patent application, the hair additive of the present invention allows significantly reducing the ammonia vapours emitted by these dyes both during storage and use. At the same time, the hair dye additive provides additional benefits such as, in particular, better coverage of hair colouring and maintenance of hair integrity.

Further features and advantages of the present invention will become clearer in the following detailed description, which is provided by way of indicative and non-limiting example with reference to the appended figures:

- Figures 1-3 show graphs concerning the variation in the amount (in ppm) of ammonia vapours released by dyes according to the invention and by comparative dyes with respect to the time (in minutes) of application to the hair,

- Figure 4 shows a graph concerning the % loss of ammonia over time (in days) for a dye according to the invention and a similar comparative additive-free dye stored in a glass jar at 40°C for 90 days.

Detailed description

The hair dye additive according to the invention relates to a particular composition adapted to be added to the hair dye or, preferably, to be mixed to the hair dye upon use thereof when the dye precursors and the oxidizer are mixed together, so as to break down the ammonia vapours that develop from mixing the aforementioned components.

The hair additive according to the invention comprises citric acid, phosphoric acid, polyethylene glycol (PEG), and at least one further acid selected from malic acid, lactic acid, succinic acid, and combinations thereof. Preferably, said further acid is made of malic acid.

Preferably, the content of citric acid is comprised between 40% and 60%, whereas the content of phosphoric acid is comprised between 10% and 20%, wherein the percentages are by weight on the total weight of the additive according to the invention. Phosphoric acid may be added to form the additive of the invention in the form of an aqueous solution thereof, in particular an 85% w/w aqueous solution.

Citric acid and phosphoric acid are able to combine with the ammonia released by mixing the hair dye precursors with the oxidizer, thus forming a soluble salt.

PEG advantageously acts as thickening and viscosity-increasing agent in the composition forming the additive and is preferably PEG 200.

Preferably, the content of PEG is comprised between 20% and 40% by weight on the total weight of the additive.

According to the present invention, the additive comprises a further acid selected from malic acid, lactic acid, succinic acid, and combinations thereof, said further acid being preferably malic acid. Preferably, the content of the at least one further acid, such as in particular malic acid, in the additive is comprised between 1% and 10%, in particular between 5% and 10%.

Advantageously, the further acid selected from the further acids above indicated and combinations thereof, in particular malic acid, acts in synergy with citric acid and phosphoric acid in the salification of ammonia, thus contributing to a further reduction in the amount of ammonia vapours released from the mixing of hair dye precursors with the oxidizer, such as hydrogen peroxide.

In particular, malic acid is an alpha-hydroxy dicarboxylic organic acid, naturally occurring in both plants and animals, including humans. Being a dicarboxylic acid, it is capable of reacting with basic substances, leading to the formation of salts, reason why it plays an important role in the salification reaction of ammonia.

More in detail, malic acid is diprotic, so it has two acid dissociation constants on a logarithmic scale: $pk1 = 3.46$ and $pk2 = 5.10$. Ammonia, on the other hand, has a basic dissociation constant $pKb = 4.74$. Since the $pK1$ value of malic acid is lower than $pKb$ of ammonia and $pK2$ is similar to $pKb$, malic acid has good salification properties with regard to ammonia in solution.

Good salification properties with regard to ammonia in solution are also observed with the use of lactic acid and succinic acid alone or in combination with malic acid.

Moreover, advantageously, the use of the further acid selected from the further acids above indicated and combinations thereof, in particular of malic acid, in the hair dye additive according to the invention provides additional benefits such as improved hair colour coverage and maintenance of hair integrity.

While not wishing to be bound to any scientific theory, it is believed that the further acid selected from the further acids above indicated and combinations thereof, in particular malic acid, is able to penetrate into the hair and create hydrogen bonds with proteins, as well as restoring electrostatic interactions in damaged or bleached hair, leading to stronger hair.

In particular, malic acid can penetrate into the hair and be absorbed at the site where water originally binds, even in extremely dry conditions, preventing water penetration and creating strong, stable hydrogen bonds with hair proteins. The formation of these strong and stable hydrogen bonds suppresses the exchange of hydrogen bonds with water, which causes breakage and weakening of the hair's structure.

The hair additive according to the invention may further comprise one or more other functional components, such as solubilisers adapted to ensure greater solubility of the additive and better applicability of the dye to the hair, emulsifiers and/or viscosity regulators adapted to adjust viscosity and ensure correct emulsification of the dye components, water, preferably deionised water, and chelants.

Suitable solubilisers comprise dimethicone meadowfoamate, dimetichone PEG-8 meadowfoamate, and mixtures thereof.

Suitable emulsifiers and/or viscosity regulators comprise glycols, such as in particular propylene glycol.

Suitable chelants comprise etidronic acid.

Preferably, the content of each of the above other components is equal to or less than 6% by weight on the total weight of the additive.

In particular, the content as a percentage by weight on the weight of the additive for dimethicone meadowfoamate is preferably comprised between 2% and 5%, more in particular between 3% and 4%.

The percentage content by weight on the weight of the additive is preferably comprised between 2% and 5% and, in particular, is comprised between 2% and 3.7%, for dimethicone PEG-8 meadowfoamate.

The percentage content by weight on the weight of the additive is preferably comprised between 2% and 6% and, in particular, comprised between 3% and 5%, for propylene glycol.

The percentage content by weight on the weight of the additive is preferably less than 1% and, in particular, is comprised between 0.3% and 0.5%, for etidronic acid.

The percentage content by weight on the weight of the additive is preferably comprised between 0.5% and 5.5% for water.

The invention also relates to a hair dye, in particular an oxidation dye, comprising dyeing precursors and an additive as above described and a method for the preparation thereof comprising the steps of:

- providing a hair dye containing dyeing precursors,
- mixing the additive above described with said dye.

The amount of additive according to the invention mixed with the hair dye may be suitably adjusted based on the amount of ammonia contained in and/or developable by the dye in use.

Preferably, the content of the additive according to the invention mixed to the hair dye is comprised between 1% and 5%, in particular between 2.5% and 4%, wherein the percentages are by weight on the total weight of the dye.

Advantageously, the addition of additive in the hair dye containing dyeing precursor substances may be performed during the industrial production of the dye. In this way, the additive counteracts a possible development of ammonia vapours throughout the entire storage period of the dye.

As for the use of the additive according to the invention above described, it should be noted that, when performing colouring, the operator prepares the dye by placing the oxidizer, such as hydrogen peroxide, and the precursors into a bowl and mixing them together, then adds the additive to the mixture of oxidizer and precursors.

Alternatively, the operator mixes the precursors with the additive in a bowl first and only afterwards adds hydrogen peroxide $H_2O_2$.

In a further alternative, the additive may be premixed with the precursors of the hair dye during the industrial production of the various shades (colours). In this way, the operator mixes the hair dye comprising the precursors and the additive directly with the oxidiser in a bowl.

Once the mixing of the three components has been completed, the dye is ready to be applied to the hair.

Therefore, the invention also comprises a new process for the preparation of a hair dye in which, as described above, precursors, oxidizer and the hair dye additive above described are reciprocally mixed.

EXPERIMENTAL PART

Example 1 - Preparation of hair dye formulations according to the invention

An additive according to the invention has been prepared by mixing citric acid, phosphoric acid in an aqueous solution concentrated to 85% by weight, malic acid, and PEG 200 in amounts expressed as percentages by weight of the additive, as indicated in Table 1 below.

Table 1

| Ingredients | % w/w |
|---|---|
| Citric acid | 50 |
| Phosphoric acid, 85% concentration | 15 |
| Malic acid | 5 |
| PEG 200 | 30 |

The resulting mixture in the viscous liquid form has been added to cream commercial hair dyes of various colours (shades) in amounts comprised between 2.5% and 4% by weight on the weight of the dye, wherein the amount of added additive has been proportionally selected to the content of dye ammonia, thus obtaining hair dye formulations according to the invention in the form of cream.

Example 2 - Ammonia vapor abatement tests

Tests were carried out to reduce ammonia vapours by means of hair dye formulations prepared according to Example 1, starting from cream commercial hair dyes containing ammonia in a different amount, which the additive of the invention was added to. The results of these tests were compared with those obtained from similar tests carried out on commercial hair dyes (comparative dyes) with the same ammonia content and not containing the additive according to the invention.

Three different colours have been chosen: 1000/8/6, which the products indicated in Table 2 below correspond to, where the same product description/numbering corresponds to the same ammonia content.

The tests were carried out by mixing the dye with hydrogen peroxide in cream of variable concentration in a plastic bowl, according to the ratios indicated in Table 2.

The bowl was then placed into a glass case that was only partially open, over whose opening an ammonia gas detector (Gas Badge Pro sold by Industrial Scientific) was placed. The gas detector detected the ammonia present in the vapours exhaled every 10 seconds throughout the entire application period.

The application times correspond to the normal application times adopted by hairdressers when using these shades.

Table 2

| Ammonia Vapour Release Analysis - Test Conditions | | | | | |
|---|---|---|---|---|---|
| Product | % Ammonia | % Additive | Hydrogen peroxide | Mix ratio | Application time |
| HAIR COLOR - 1000 + Additive | 3.25 | 4 | 40 vol (12% w/w) | 10 g dye + 20 g hydrogen peroxide | 45 min |
| HAIR COLOR - 1000 (Comp.) | 3.25 | - | 40 vol (12% w/w) | 10 g dye + 20 g hydrogen peroxide | 45 min |
| INVISIBLE COLOR - 1000 (Comp.) | 3.25 | - | 40 vol (12% w/w) | 10 g dye + 20 g hydrogen peroxide | 45 min |
| | | | | | |
| HAIR COLOR - 8 + Additive | 2.15 | 3 | 30 vol (9% w/w) | 10 g dye + 15 g hydrogen peroxide | 30 min |
| MY COLOR - 8 (Comp.) | 2.15 | - | 30 vol (9% w/w) | 10 g dye + 15 g hydrogen peroxide | 30 min |
| INVISIBLE COLOR - 8 (Comp.) | 2.15 | - | 30 vol (9% w/w) | 10 g dye + 15 g hydrogen peroxide | 30 min |
| | | | | | |
| HAIR COLOR - 6 + Additive | 1.85 | 3 | 20 vol (6% w/w) | 10 g dye + 15 g hydrogen peroxide | 30 min |

(continued)

| Ammonia Vapour Release Analysis - Test Conditions | | | | | |
|---|---|---|---|---|---|
| Product | % Ammonia | % Additive | Hydrogen peroxide | Mix ratio | Application time |
| MYCOLOR - 6 (Comp.) | 1.85 | - | 20 vol (6% w/w) | 10 g dye + 15 g hydrogen peroxide | 30 min |
| INVISIBLE COLOR - 6 (Comp.) | 1.85 | - | 20 vol (6% w/w) | 10 g dye + 15 g hydrogen peroxide | 30 min |

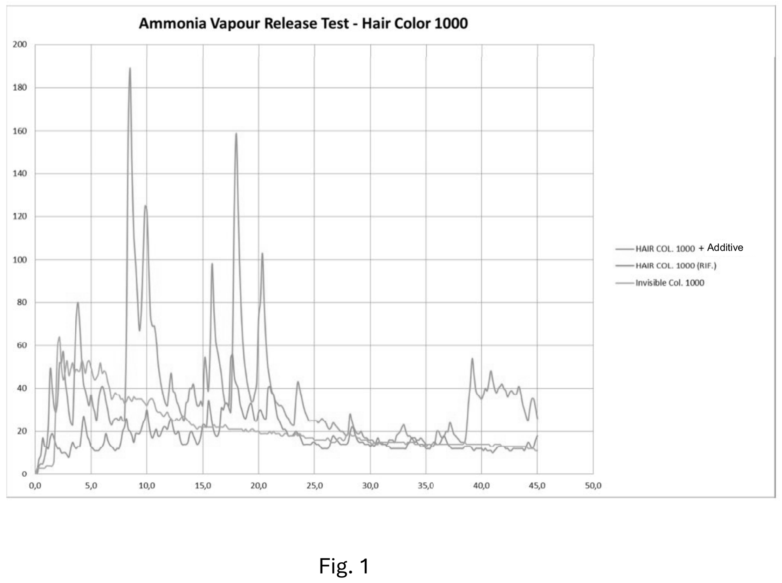

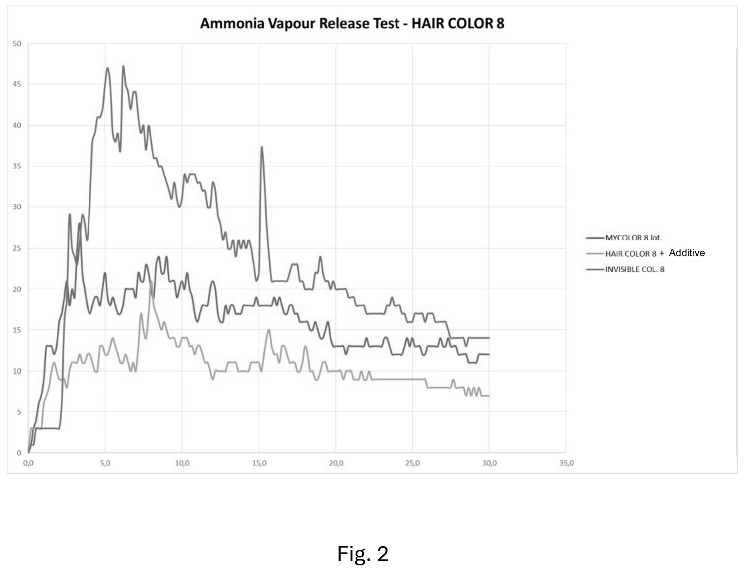

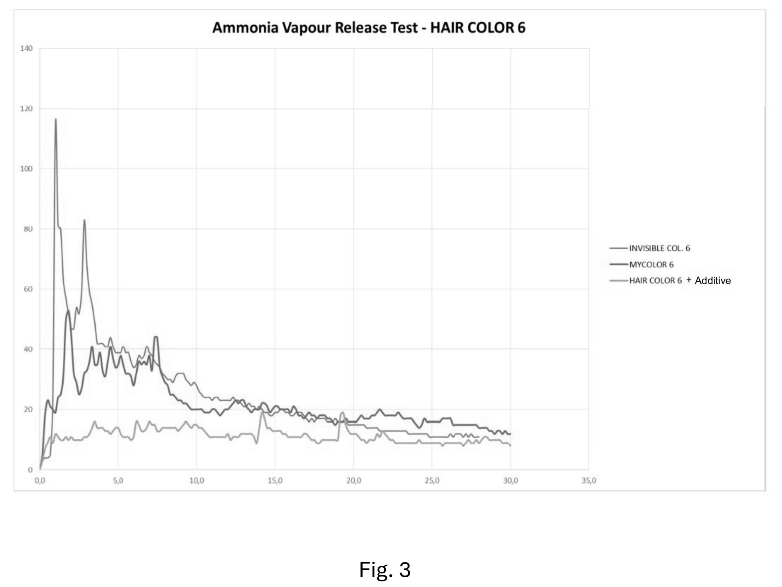

Figures 1-3 show graphs relating to the variation in the amount of ammonia vapours released by the dyes according to the invention and the comparative dyes with respect to the exposure time (application) on the hair.

The effectiveness of the additive added to the dyes according to the invention in salifying ammonia and retaining the ammonia vapours with respect to additive-free dyes is well visible from the graphs reported in Figures 1-3 and even from the maximum and average values of the ammonia vapours (ppm), which are less in the dyes containing the additive of the invention, as clear from Table 3 below.

Table 3

| Ammonia Vapour Release Analysis | | | | |
|---|---|---|---|---|
| Product | % Ammonia | % Additive | Max - NH3 values (ppm) | Average - NH3 Values (ppm) |
| HAIR COLOR - 1000 + Additive | 3.25 | 4 | 56 | 17.4 |
| HAIR COLOR - 1000 (Comp.) | 3.25 | - | 189 | 37.3 |
| INVISIBLE COLOR - 1000 (Comp.) | 3.25 | - | 64 | 21.9 |
| | | | | |
| HAIR COLOR - 8 + Additive | 2.15 | 3 | 21 | 10.3 |
| MY COLOR - 8 (Comp.) | 2.15 | - | 28 | 15.9 |
| INVISIBLE COLOR - 8 (Comp.) | 2.15 | - | 47 | 23.3 |
| | | | | |
| HAIR COLOR - 6 + Additive | 1.85 | 3 | 19 | 11.3 |
| MYCOLOR - 6 (Comp.) | 1.85 | - | 53 | 21.8 |
| INVISIBLE COLOR - 6 (Comp.) | 1.85 | - | 115 | 24.1 |

The ammonia exhalation peak normally occurs within the first 10 minutes of exposure (see graphs in Figures 1-3), and in the dyes (shades) containing the additive according to the invention a reduction of this peak up to 83% was found with respect to the similar comparative dyes compared with tests of the additive of the invention.

In terms of average exhalation during the application time, on the other hand, in the dyes containing the additive of the invention a reduction in ammonia emissions up to 55% was found with respect to the analogous comparative dyes compared with tests of the additive of the invention.

Example 3 - Tests on ammonia salification

Further tests were carried out on the ability of salifying ammonia by means of the additive according to the invention, monitoring the ammonia content of two dye samples with the same ammonia content, one containing the additive according to the invention prepared according to Example 1 in an amount of 4% by weight, and the other one additive-free. Both dyes were placed into a glass jars in a thermostatic oven at 40°C for 90 days to stabilise.

The ammonia content was determined by means of Kjeldahl distillation, capture with boric acid, and subsequent titration with sulphuric acid.

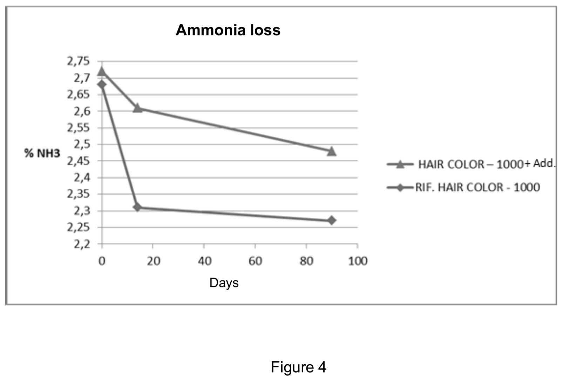

Figure 4 shows a graph relating to the % loss of ammonia over time (in days) of the above dyes during the storage period in the respective glass jar.

It can be seen from Figure 4 and from the results reported in Table 4 below that the loss of ammonia ($\Delta\%$)

compared to the initial content is overall lower and more gradual in the dye containing the additive according to the invention. This indicates that ammonia is better retained in salified form within the dye matrix.

Table 4

| Ammonia Content Analysis % | | | | | | |
|---|---|---|---|---|---|
| | 0 days | 14 days | | 90 days | |
| Product | %NH3 | %NH3 | Δ% | %NH3 | Δ% |
| HAIR COLOR - 1000 + Additive | 2.72 | 2.61 | -4,0 | 2.48 | -8.8 |
| HAIR COLOR - 1000 (Comp.) | 2.68 | 2.31 | -13.8 | 2.27 | -15.3 |

Example 4 - White hair coverage tests

Colouring tests were carried out on white hair in double on different dyes containing the additive according to the invention prepared according to Example 1. These tests were compared with those carried out on comparative dyes of equal composition and dye and ammonia content but without the additives according to the invention.

The dyes of the invention and the comparative dyes were applied on yak hair, representative of the 100% white hair and natural hair base 6 (dark blonde) with 70% grey, and instrumental measurements were carried out through EOPTIS CLM-194 colorimeter after 48 hours from application.

Colorimetric measurements were carried out on yak base (100% white hair) where the chromatic differences are more highlighted, whereas base 6 with 70% grey was used for a further visual comparison.

As representative parameter of the white hair coverage, the overall colour variation "ΔE", calculated with respect to the undyed yak base (reference) was used. Higher ΔE values indicate greater coverage of grey hair.

Parameter ΔE*ab, i.e. the colour difference calculated with respect to a reference, is defined as the distance between two points in the CIELAB 1976 colour space according to the following formula:

$$\Delta E^*_{ab} = \sqrt{(L^*_1 - L^*_0)^2 + (a^*_1 - a^*_0)^2 + (b^*_1 - b^*_0)^2}$$ where the values "L", "a", "b" represent the coordinates of the colour in the three-dimensional space CIELAB, specifically:

L1 = Brightness (black to white) of the sample;

a1 = colour coordinate (from green to red) of the sample;

b1 = colour coordinate (from blue to yellow) of the sample;

L0 = Brightness (black to white) of the reference;

a0 = colour coordinate (from green to red) of the reference;

b0 = colour coordinate (from blue to yellow) of the reference.

The list of the tested colours and of the results is reported in Table 5.

Table 5

| Colorimetric analyses | | |
|---|---|---|
| Product | % Additive | ΔE |
| 7.0 + Additive | 3 | 50.24 |
| 7.0 (Comp.) | - | 48.48 |
| 6.0 + Additive | 3 | 55.85 |
| 6.0 (Comp.) | - | 55.03 |
| 7.00 + Additive | 3 | 53.81 |
| 7.00 (Comp.) | - | 52.57 |
| 6.00 + Additive | 3 | 56.07 |

(continued)

| Colorimetric analyses | | |
|---|---|---|
| Product | % Additive | ΔE |
| 6.00 (Comp.) | - | 54.36 |
| 7.3 + Additive | 3 | 45.30 |
| 7.3 (Comp.) | - | 41.14 |
| 8.31 + Additive | 3 | 31.48 |
| 8.31 (Comp.) | - | 28.24 |

As can be seen, in all of the tested cases the dyes containing the additive according to the invention have higher ΔE values, greater coverage index for white hair, with respect to the comparative dyes of the same composition but additive-free. This result is also confirmed visually from the photos of the results on a strand of hair (not shown).

Example 5 - Stability and Compatibility Tests

Stability tests were carried out in a glass jar and compatibility tests were carried out with aluminium tube packaging coated internally with epoxyphenolic resin on two dyes containing the additive according to the invention prepared according to Example 1. The tested dyes are 1000: super lightening with high ammonia content, and 1: black with high dye content.
The 1000 dye contains 3.25% salified ammonia with 4% additive according to the invention, whereas 1 dye contains 1.55% salified ammonia with 2.5 % additive of the invention.
Both tests were carried out in parallel at temperatures of 4°C, 20°C and 40°C, for a duration of 12 weeks.
During the stability tests the following parameters were monitored on a bi-weekly basis: aspect, colour, smell, pH, viscosity (cPs), and colour result on strand.
Compatibility tests were carried out in parallel with tubes from three different suppliers, monitoring the appearance of the tube twice a week.
At the end of the stability tests, no anomalies or deviations in the monitored chemical-physical parameters were found.
At the end of the compatibility tests, no anomalies were found in the packaging, such as corrosion, colour changes, deformation, or swelling.

Example 6 - Tests on a model

Application tests were carried out on a model to assess the effects of the additive according to the invention on the health of the hair and to confirm the colouring results.
The tests were carried out by dividing the hair into two vertical sections. A dye mixture (dye + oxidiser cream) of various shades was applied to the right section, in particular from the natural series ranging from black to blonde, without addition of the additive according to the invention (reference), whereas on the left side, a dyeing mixture of the same shade was applied, also containing the additive of the invention according to Example 1 in a variable amount 2.5 to 4%.
The parameters (effects) under assessment were observed on dry hair as per the diagram reported in Table 6 below.

Table 6

| | Effects | Evaluation method | Judgment |
|---|---|---|---|
| 1 | Improved brightness | Visual | Better / equivalent / worse than the reference |
| 2 | Improved hair nourishment | Visual / Tactile | Better / equivalent / worse than the reference |
| 3 | Improved hair softness | Tactile | Better / equivalent / worse than the reference |
| 4 | Colour result and white hair coverage | Visual | Better / equivalent / worse than the reference |

The tests were carried out on 10 healthy subjects.
The above effects are considered confirmed when the number of "Better than the reference" ratings is greater than 5.

The results of the test are reported in Table 7 below.

Table 7

|   | Effects | Best | Equivalent | Worst |
|---|---|---|---|---|
| 1 | Improved brightness | 9/10 | 1/10 | 0/10 |
| 2 | Improved hair nourishment | 8/10 | 2/10 | 0/10 |
| 3 | Improved hair softness | 8/10 | 2/10 | 0/10 |
| 4 | Colour result and white hair coverage | 9/10 | 1/10 | 0/10 |

As can be seen, all of the effects of improved hair brightness, improved hair nourishment, improved hair softness, improved colour results, and white hair coverage are confirmed by the use of the dye containing the additive according to the invention.

In light of the above, the hair dye additive according to the invention and the dye containing said additive achieve the intended goals and offer significant advantages over the prior art.

A first important advantage lies in the fact that, thanks to the additive above described, hair dye vapours and, in particular, ammonia vapours, are greatly reduced.

Indeed, the simultaneous presence in the additive of the invention of citric acid, phosphoric acid, and at least one further acid selected from lactic acid, succinic acid, and malic acid, in particular malic acid, allows the ammonia present in the dye to be effectively salified and, therefore, not to disperse into the air in the form of gas, both during the storage of the dye and during its use in combination with an oxidizer such as hydrogen peroxide.

Another advantage lies in the fact that, thanks to the lower presence of free ammonia in solution, the mixing of oxidizer and precursors does not determine the development of irritating and smelly gases and/or vapours in the atmosphere. This advantage is particularly important for hairdressers and customers since the absence of said vapours allows a non-smelly environment.

A further important advantage lies in the fact that the dye containing the additive according to the invention achieves better performances deriving from the application on the hair, in particular in terms of improved hair nourishment, improved hair softness and improved colour results and white hair coverage, while preserving the integrity of the hair.

A further advantage lies in the fact that the dye containing the additive according to the invention is sufficiently stable and compatible with the common packaging used for cream dyes, such as aluminium tubes internally coated with epoxy-phenolic resin, therefore being able to be stored for sufficiently long periods of at least 3 months at 40°C, equivalent to over a year at room temperature without any significant degradation and/or alterations to the packaging occurring.

**Claims**

1.    A hair dye additive comprising citric acid, phosphoric acid, polyethylene glycol (PEG), and at least one further acid selected from malic acid, lactic acid, succinic acid and combinations thereof.

2.    The additive according to claim 1, wherein said at least one further acid is malic acid.

3.    The additive according to claim 1 or 2, wherein the content of citric acid is comprised between 40% and 60% and the content of phosphoric acid is between 10% and 20%, wherein the percentages are by weight of the total weight of the additive.

4.    The additive according to any one of the preceding claims, wherein the content of PEG, preferably PEG 200, is comprised between 20% and 40% by weight of the total weight of the additive.

5.    The additive according to any one of the preceding claims, wherein the content of said at least one further acid, preferably malic acid, is comprised between 1% and 10%, preferably between 5 % and 10%, by weight of the weight of the additive.

6.    The additive according to any one of the preceding claims comprising at least one further component selected from dimethicone meadowfoamate, dimethicone PEG-8 meadowfoamate, water, in particular deionized water, and etidronic acid, wherein the content of each of said components is preferably equal to or lower than 6% by weight on the weight of the additive.

7.  An oxidation hair dye comprising dyeing precursors and an additive according to any one of the preceding claims.

8.  The hair dye according to claim 7, **characterized in that** it is substantially in the form of a cream.

9.  A method for the preparation of a hair dye according to claim 7 or 8, the method comprising the steps of:

    - providing an oxidation hair dye containing dyeing precursors,
    - mixing an additive according to any one of claims 1 to 6 with said dye.

10. The method according to claim 9, wherein said additive is mixed with said dye in an amount comprised between 1% and 5%, preferably between 2.5% and 4%, wherein the percentages are by weight of the total weight of the dye.

11. The method for the preparation of a mixture for hair dyeing comprising the step of mixing an oxidation hair dye containing dyeing precursors, an oxidizer, and a hair-dye additive according to any one of claims 1 to 6.

12. The method according to claim 11, wherein said additive is premixed with said dye containing dyeing precursors and said oxidizer is added to the mixture of the dye and the additive.

Fig. 1

Fig. 2

Fig. 3

Figure 4

Europäisches
Patentamt

European
Patent Office

Office européen
des brevets

## EUROPEAN SEARCH REPORT

Application Number

EP 25 20 9097

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE GNPD [Online] MINTEL; 10 August 2022 (2022-08-10), anonymous: "Body Wash", XP093273858, Database accession no. 9808114 * abstract * | 1,6 | INV. A61K8/24 A61K8/365 A61K8/86 A61Q5/10 |
| X | DATABASE GNPD [Online] MINTEL; 1 December 2014 (2014-12-01), anonymous: "Hokkaido Nourish Milk Body Foam", XP093273862, Database accession no. 2804403 * abstract * | 1,2,6 | |
| X | CN 116 034 997 B (RINGPU TIANJIN BIO PHARM CO LTD) 13 September 2024 (2024-09-13) * examples 1-5, 8 * | 1,2,5,6 | |
| Y,D | EP 2 873 411 A1 (GIANNICO PAOLO [IT]; ARCELLI ANTONIO [IT]; DANIELLI MARCO [IT]) 20 May 2015 (2015-05-20) * paragraphs [0011], [0012]; claims * | 1-12 | TECHNICAL FIELDS SEARCHED (IPC) A61K A61Q |
| Y | US 2018/338894 A1 (NÖCKER BERND [DE] ET AL) 29 November 2018 (2018-11-29) * paragraph [0007] * * paragraph [0033]; claim 2 * * paragraphs [0081], [0086]; examples 1-8 * | 1-12 | |
| Y | CN 106 890 557 A (JIANGSU REFONTECH IND CO LTD) 27 June 2017 (2017-06-27) * paragraph [0007]; claims; examples 1-7 * | 1-12 | |

-/--

The present search report has been drawn up for all claims

| Place of search | Date of completion of the search | Examiner |
|---|---|---|
| Munich | 2 March 2026 | Perrone Dunet, S |

page 1 of 2

Europäisches
Patentamt

European
Patent Office

Office européen
des brevets

## EUROPEAN SEARCH REPORT

**Application Number**

EP 25 20 9097

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CN 117 618 310 B (FOSHAN XIZHUANG COSMETICS CO LTD) 13 September 2024 (2024-09-13) * paragraphs [0005], [0007]; claim 1 * ----- | 1-12 | |
| Y | US 2021/015729 A1 (MATSUTANI AKIRA [JP] ET AL) 21 January 2021 (2021-01-21) * paragraph [0010]; claims 1, 5; examples 1-7 * ----- | 1-12 | |
| | | | TECHNICAL FIELDS SEARCHED (IPC) |

The present search report has been drawn up for all claims

| Place of search | Date of completion of the search | Examiner |
|---|---|---|
| Munich | 2 March 2026 | Perrone Dunet, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 20 9097

This annex lists the patent family members relating to the patent documents cited in the above-mentioned European search report.
The members are as contained in the European Patent Office EDP file on
The European Patent Office is in no way liable for these particulars which are merely given for the purpose of information.

02-03-2026

| Patent document cited in search report | | Publication date | Patent family member(s) | | Publication date |
|---|---|---|---|---|---|
| CN 116034997 | B | 13-09-2024 | NONE | | |
| EP 2873411 | A1 | 20-05-2015 | EP | 2873411 A1 | 20-05-2015 |
| | | | ES | 2838299 T3 | 01-07-2021 |
| US 2018338894 | A1 | 29-11-2018 | AU | 2016318285 A1 | 12-04-2018 |
| | | | US | 2018338894 A1 | 29-11-2018 |
| | | | WO | 2017041909 A1 | 16-03-2017 |
| CN 106890557 | A | 27-06-2017 | NONE | | |
| CN 117618310 | B | 13-09-2024 | NONE | | |
| US 2021015729 | A1 | 21-01-2021 | JP | 6682567 B2 | 15-04-2020 |
| | | | JP | 2019178104 A | 17-10-2019 |
| | | | US | 2021015729 A1 | 21-01-2021 |
| | | | WO | 2019187232 A1 | 03-10-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2873411 A